(19) Europäisches Patentamt / European Patent Office / Office européen des brevets

(11) **EP 2 994 099 B1**

(12) **FASCICULE DE BREVET EUROPEEN**

(45) Date de publication et mention de la délivrance du brevet:
**16.01.2019   Bulletin 2019/03**

(21) Numéro de dépôt: **14726425.3**

(22) Date de dépôt: **06.05.2014**

(51) Int Cl.:
*A61K 8/58* (2006.01)   *A61K 8/67* (2006.01)
*A23L 33/10* (2016.01)   *A23L 33/15* (2016.01)
*A61K 8/36* (2006.01)   *A61Q 5/00* (2006.01)
*A61K 8/92* (2006.01)   *A23L 33/155* (2016.01)
*A23L 33/16* (2016.01)   *A61K 8/46* (2006.01)
*A23L 33/175* (2016.01)

(86) Numéro de dépôt international:
**PCT/IB2014/061246**

(87) Numéro de publication internationale:
**WO 2014/181258 (13.11.2014 Gazette 2014/46)**

(54) **ASSOCIATION D'ACIDE PETROSELINIQUE ET DE TAURINE POUR UNE ADMINISTRATION PAR VOIE ORALE POUR LUTTER CONTRE LE VIEILLISSEMENT DES CHEVEUX**

KOMBINATION AUS PETROSELINSÄURE UND TAURIN ZUR ORALEN VERABREICHUNG ZWECKS BEKÄMPFUNG DER ALTERUNG DER HAARE

COMBINATION OF PETROSELINIC ACID AND TAURINE FOR ORAL ADMINISTRATION FOR HAIR AGING CONTROL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **07.05.2013   FR 1354193**

(43) Date de publication de la demande:
**16.03.2016   Bulletin 2016/11**

(73) Titulaire: **NUTRICOS Technologies**
**92117 Clichy Cedex (FR)**

(72) Inventeurs:
• **MAHE, Yann**
  **F-91700 Sainte Genevieve Des Bois (FR)**
• **BRU, Carole**
  **F-92400 Courbevoie (FR)**
• **CASTIEL, Isabelle**
  **F-NICE 06200 (FR)**
• **GUENICHE, Audrey**
  **F-92500 Rueil Malmaison (FR)**

(74) Mandataire: **Nony**
**11 rue Saint-Georges**
**75009 Paris (FR)**

(56) Documents cités:
**EP-A1- 1 013 178      EP-A1- 1 932 509
WO-A2-02/07700      WO-A2-2004/000293
WO-A2-2008/071897      CN-A- 102 293 312**

• **FRDRIC DESTAILLATS ET AL: "Identification of 6-monounsaturated fatty acids in human hair and nail samples by gas-chromatographymass-spectrometry using ionic-liquid coated capillary column", JOURNAL OF CHROMATOGRAPHY, ELSEVIER SCIENCE PUBLISHERS B.V, NL, vol. 1218, no. 52, 21 octobre 2011 (2011-10-21), pages 9384-9389, XP028338533, ISSN: 0021-9673, DOI: 10.1016/J.CHROMA.2011.10.095 [extrait le 2011-11-15]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

La présente invention se rapporte au domaine du soin des fibres kératiniques, plus particulièrement des cheveux. En particulier, elle vise à proposer une association utile pour améliorer la qualité de la chevelure et pour lutter contre le vieillissement des fibres kératiniques, et plus particulièrement capillaires, provoquant en particulier un affinement des fibres kératiniques ou plus globalement des cheveux.

Comme cela apparaitra par la suite, il est plus particulièrement question dans le cadre de la présente invention de la lutte contre le vieillissement des cheveux chez l'homme de plus de 30 ans ou chez la femme.

Les cheveux sont produits dans des follicules pileux formés de gaines épithéliales d'origine épidermique et d'un bulbe pileux contenant des kératinocytes bulbaires en état de division permanent pendant les phases de croissances du cheveu. Le cheveu est constitué principalement de 85-90 % de protéines.

Avoir des cheveux sains et vigoureux tout au long de la vie est recherché par la plupart des femmes et des hommes.

Ainsi c'est un premier objectif de l'invention que de proposer une solution aux hommes et femmes de tout âge pour améliorer la qualité de leur chevelure.

En particulier, les hommes de plus de 30 ans et les femmes sont soumis à un vieillissement de leurs fibres kératiniques, en particulier capillaires. Ils peuvent ainsi présenter une alopécie liée au vieillissement de la chevelure qui se caractérise notamment par un affinement et une perte de matière globale de la chevelure et d'un éclaircissement progressif.

Ceci se distingue par certains aspects de l'alopécie dite androgénétique dont la transmission est par définition héréditaire, dont la progression est rapide et affecte initialement le front et les tempes et, en raison du rôle des androgènes, se signale très tôt après la puberté, principalement chez l'homme jeune.

De récents travaux ont mis en évidence chez les individus plus âgés, et indifféremment chez les hommes et les femmes, une forme d'alopécie plus longue à s'installer dans le temps mettant en jeu la contribution d'une forme latente et silencieuse d'inflammation (Pro-inflammatory cytokine cascade in human plucked hair Mahé *et al.* Skin Pharmacol 1996, 366-375), dite à bas bruit, et appelée microinflammation (Mahé et al. Androgenetic alopecia and micro inflammation Int. J. Dermatol., 2000, 39, 576-584 et par Trueb, Clinical intervention in aging hair, 2006:1(2) 121-129) comme contributeur de l'involution progressive des follicules pileux et de la miniaturisation du follicule pileux.

Ce phénomène dit de microinflammation du follicule pileux a été identifié et décrit dans Mahé et al. (International Journal of Dermatology, 2000, 39, 576-584) comme étant une inflammation lente, subtile et indolore, qui n'entraîne ni rougeurs, démangeaisons ou chaleurs, ni aucun des troubles d'ordre thérapeutique habituellement associés à une inflammation du scalp.

Bien que pouvant faire intervenir des infiltrats cellulaires inflammatoires lymphocytaires ou monocytaires périfolliculaires, elle se distingue nettement de l'inflammation dite classique, pouvant avoir lieu au niveau du derme ou du scalp, qui se manifeste de manière bien plus gênante, voire douloureuse, et qui a des conséquences bien plus destructrices sur le cuir chevelu et sur la fibre capillaire. Notamment, elle se distingue de l'*alopecia areata* liée à un trouble immuno-inflammatoire établi et qui aboutit à une chute massive et parfois localisée et souvent spontanément réversible des cheveux, affectant indifféremment des hommes, des femmes et des enfants, ce qui permet d'exclure a priori la contribution des androgènes dans sa physiopathologie.

La microinflammation à bas bruit contribue au contraire à un vieillissement plus progressif et intrinsèque du scalp et des cheveux. Comme indiqué précédemment, la microinflammation périfolliculaire et folliculaire selon l'invention peut entrainer, outre un affinement des cheveux, une chute des cheveux, et aboutir à une alopécie plutôt diffuse voire centrée sur le vertex, en particulier chez l'homme de plus de 30 ans ou chez la femme, par opposition à l'alopecie androgenetique qui démarre au niveau du vertex et du front et affecte principalement les hommes jeunes.

Le vieillissement du cheveu, caractérisé par cette microinflammation, est en effet un phénomène naturel qui se manifeste progressivement et plus ou moins tôt selon les individus. Cette microinflammation est difficilement perceptible en surface et a notamment pour conséquence le déclenchement de perturbations dermiques périfolliculaires conduisant à un épaississement de la gaine interne du cheveu et à sa miniaturisation progressive au fur et à mesure des cycles successif de régénération d'un nouveau follicule fonctionnel. On constate ainsi un affinement du cheveu, un cheveu moins épais, moins résistant à la traction et à la casse, et une diminution du volume de la chevelure globale. En outre, le scalp devient visible par endroits avec un net dégarnissement du vertex, sans pour autant systématiquement affecter fortement les tempes ou le front. Parfois même, cette alopécie diffuse, tardive et à évolution lente peut prendre le relais ou renforcer et accentuer les effets d'une phase initiale d'alopécie androgénétique. En outre, le vieillissement du cheveu peut également se caractériser par une usure et une altération de la fibre capillaire se traduisant par des cheveux dits fragilisés/sensibilisés, ou bien encore des cheveux secs.

Ainsi, il apparait clairement que cette microinflammation périfolliculaire, qui est représentative du vieillissement intrinsèque du bulbe et des fibres capillaires, n'est à l'origine que de troubles cosmétiques, et notamment de ceux énoncés précédemment. Contrairement à l'inflammation dite classique, dont les troubles associés se situent dans le domaine thérapeutique, la prévention et/ou le traitement de la microinflammation cliniquement silencieuse et à bas bruit du follicule pileux, et ainsi des troubles qui lui sont associés, se situent uniquement dans le domaine cosmétique.

Il existe donc également un besoin de disposer d'actifs, voire d'une association d'actifs, aptes à prévenir et/ou lutter contre la microinflammation des follicules pileux.

De nombreuses compositions cosmétiques contenant des acides aminés, en particulier la glutamine, l'arginine et la cystéine, ou des peptides et/ou de la taurine existent pour le soin du cuir chevelu.

A titre d'exemple, le document WO 2004/000293 décrit l'utilisation de la taurine et/ou de l'hypotaurine et/ou leurs sels acceptables pour la préparation d'une composition orale utile dans le traitement et la prévention du vieillissement de l'unité pilosébacée et/ou de l'alopécie.

En outre, le document WO 2008/071897 décrit une composition comprenant de l'acide pétrosélinique pour l'application topique sur le cheveu, pour le traitement ou la prévention de leur altération, et pour améliorer la qualité des fibres kératiniques.

Pour autant, les consommateurs sont toujours à la recherche d'actifs ou d'association d'actifs plus efficaces pour améliorer la qualité de leur chevelure. En outre, il n'existe pas à ce jour d'actifs spécifiquement adaptés à la prévention et à la lutte contre la microinflammation à bas bruit du follicule pileux et contre l'affinement progressif des cheveux, en particulier chez l'homme de plus de 30 ans ou chez la femme.

La présente invention propose de répondre à ce besoin en proposant une association d'acide pétrosélinique avec de la taurine administrée par voie orale.

Comme illustré dans les exemples de la présente demande, les inventeurs ont en effet constaté qu'une association d'actifs conforme à l'invention s'avère apte à augmenter de façon synergique la quantité de lipoxine A4. La lipoxine A4 appartient à la famille des résolvines. Cette famille de composés naturellement produits par l'organisme agit de façon complémentaire aux agents anti-inflammatoires classiques en élevant le seuil de déclenchement d'une réponse inflammatoire dite classique dermatologiquement, et plus particulièrement pour élever le seuil d'apparition des signaux de cette inflammation classique, à savoir les rougeurs, les douleurs et la chaleur.

L'invention a donc pour objet principal l'utilisation cosmétique par voie orale d'une association d'actifs comprenant de l'acide pétrosélinique et de la taurine, pour prévenir et/ou lutter contre la microinflammation à bas bruit des follicules pileux, permettant d'améliorer la qualité de la chevelure, en particulier chez l'homme de plus de 30 ans ou chez la femme.

De manière préférée selon l'invention, l'utilisation cosmétique par voie orale d'une association d'actifs comprenant de l'acide pétrosélinique et de la taurine est caractérisée en ce que l'amélioration de la qualité de la chevelure comprend l'amélioration de la brillance de la chevelure et/ou l'amélioration de la coiffabilité de la chevelure, et/ou de sa tenue et de son maintien et/ou la diminution de la chute des cheveux et/ou l'amélioration de la croissance de la fibre capillaire et/ou l'amélioration du volume de la chevelure et/ou l'amélioration de la qualité de la fibre capillaire.

De manière encore préférée, l'amélioration du volume de la chevelure comprend l'augmentation et/ou le maintien du diamètre de la fibre capillaire, et/ou l'augmentation de la densité, et/ou la limitation de l'affinement de la fibre capillaire.

De manière également préférée, l'amélioration de la qualité de la fibre capillaire comprend améliorer la résistance à la traction des cheveux, et/ou au coiffage et/ou à la mise en forme des cheveux, et/ou prévenir et/ou lutter contre les cheveux mous et/ou cassants et/ou ternes et/ou fourchus et/ou fragilisés et/ou sensibilisés et/ou secs, et/ou améliorer la douceur et/ou la vigueur des fibres capillaires.

La présente invention vise en outre l'utilisation cosmétique par voie orale d'une association d'acide pétrosélinique et de la taurine pour lutter contre le vieillissement du cheveu chez l'homme de plus de 30 ans ou chez la femme.

La présente invention vise en particulier l'utilisation cosmétique, par voie orale, d'une association d'acide pétrosélinique et de taurine pour prévenir et/ou lutter contre la microinflammation à bas bruit des follicules pileux et/ou du scalp, en particulier des follicules pileux.

Par vieillissement du cheveu, on entend désigner selon l'invention un changement d'aspect de la fibre (cheveu fin, terne, sans tenue (mou), sans éclat), des cheveux qui ont tendance à chuter, qui se renouvellent moins vite, dont le réseau de collagène qui les soutient est altéré par la libération importante de collagénase et la désorganisation de son réseau, dont les parties dermique et épithéliale se rigidifient, dont la synthèse de sébum se réduit, entraînant une sécheresse du cuir chevelu et augmentant d'autant l'aspect terne et sans éclat du cheveu, dont l'épiderme et le derme du scalp sont aussi soumis à une désorganisation du réseau de collagène ce qui rigidifie le derme autour du follicule pileux, entraînant des effets négatifs pour les follicules résidents et l'implantation de nouveaux cheveux.

On entend également par vieillissement du cheveu une diminution de la densité capillaire, ainsi que du diamètre des cheveux, se traduisant par une diminution marquée ou diffuse de la couverture du scalp.

Par affinement des cheveux, on entend désigner selon l'invention une diminution du diamètre des cheveux en dessous de 40 $\mu$m. Le diamètre des cheveux peut-être avantageusement mesuré à l'aide du Trichoscan, équivalent automatisé du Trichogramme, où l'oeil humain a été remplacé par un logiciel d'analyse d'image (Gasmueller, 2009). Par ailleurs, en dessous de 40 $\mu$m, les cheveux sont difficilement visibles à l'oeil nu. L'affinement des cheveux pourra donc être perçu. Un seuil de 5 % pourra être considéré comme significatif.

Par pousse de cheveux épais, on entend désigner, selon l'invention, la pousse de cheveux avec un diamètre supérieur à 40 $\mu$m, mesurable par le bais du Trichoscan, mais aussi facilement visibles à l'oeil nu.

Par augmenter la densité capillaire, on entend selon l'invention un plus grand nombre de cheveux par $cm^2$. La densité des cheveux est également mesurable avec le Trichoscan. Avec cet appareil, une densité <280 cheveux par $cm^2$ est considérée comme une densité faible. Une augmentation de 5 % de la densité peut-être considérée comme cliniquement significative et visible.

Par prévenir la chute des cheveux, on entend, selon l'invention, prévenir une diminution du % de cheveux en phase télogène, mesurable avec le Trichoscan, en se basant sur le fait que les cheveux en phase télogène ne poussent plus contrairement aux cheveux en phase anagène. Une diminution de 5 % du nombre de cheveux en phase télogène peut être considérée comme significative.

Par prévenir la chute des cheveux, on entend également, selon l'invention, prévenir une diminution du % de cheveux en phase catagène, mesurable à l'aide du Trichogramme, ou par recueil de cheveux au coiffage ou après la douche en se basant sur le fait que les cheveux en phase catagène sont le reflet visible des cheveux précédemment engagés en phase d'arrêt de la croissance (phase telogène) et représentent la chute telle que réellement perçue par un individu affecté par une chute anormalement élevée de cheveux. Une diminution de 5 % du nombre de cheveux en phase catagène peut être considérée comme significative.

Par augmenter le volume de la chevelure globale, on entend désigner selon l'invention une augmentation du diamètre des cheveux associée à une diminution de l'hétérogénéité de leurs diamètres et à une augmentation du nombre de cheveux par $cm^2$.

Par prévenir et/ou lutter contre les cheveux mous et/ou cassants et/ou secs et/ou fragilisés et/ou ternes et/ou fourchus, on entend désigner selon l'invention une amélioration globale de la structure de la tige pilaire, et notamment de la cuticule, couche la plus externe du cheveu.

Par améliorer la douceur des cheveux, on entend désigner selon l'invention une amélioration de l'état des écailles de la cuticule et plus particulièrement de leur cohésion. Des écailles disjointes rendent les cheveux rêches au toucher.

Par améliorer la résistance à la traction des cheveux et la vigueur des fibres capillaires, on entend designer selon l'invention une amélioration de la solidité des cheveux qui peut se mesurer par le test à la traction. Cette mesure de détermination des propriétés mécaniques en traction du cheveu est réalisée à l'aide d'un outil commercial, le MTT600 (mini Tensile Tester) de la société Dia Stron. Une augmentation de 5 % de la force nécessaire pour rompre le cheveu est considérée comme significative.

Par la coiffabilité de la chevelure, on entend désigner selon l'invention un cheveu facile à peigner et/ou brosser. Les écailles disjointes ou endommagées s'accrochent avec les écailles des cheveux voisins provoquant l'emmêlement de la chevelure, ce qui favorise ensuite l'apparition de noeuds rendant le coiffage plus difficile.

Par coiffabilité, on entend également une meilleure tenue de la coiffure après pose de bigoudis, de brushing, de défrisage ou frisage.

Par brillance de la chevelure, on entend désigner selon la présente invention une moindre usure et/ou une réparation des écailles de la cuticule pour homogénéiser la surface du cheveu et favoriser la réflexion de la lumière.

De manière préférentielle, les différents paramètres tels que définis ci-avant pourront être évalués par des hommes et/ou des femmes au moyen d'un questionnaire à choix multiples dans le cadre d'une étude observationnelle qui pourra être réalisée chez des dermatologues. Un effet sera considéré comme significatif à partir du moment où au moins 50 % des hommes et/ou des femmes auront perçu un effet positif.

Sont particulièrement concernées par les utilisations et procédés selon l'invention les hommes de plus de 30 ans et les femmes.

Par « homme », on entend, au sens de la présente invention, la population masculine humaine.

L'invention a également pour objet la mise en oeuvre d'une association d'actifs conforme à l'invention sous forme de complément alimentaire.

L'invention a de plus pour objet une composition cosmétique destinée à une administration par voie orale ou un complément alimentaire contenant une association d'acide pétrosélinique, de taurine, zinc, ou de l'un de ses sels, de préférence complexé par un ou plusieurs (poly)hydroxyacides, de préférence le gluconate de zinc, l'acide pétrosélinique étant présent dans une teneur comprise entre 20 % et 70 % en poids par rapport au poids total de ladite association d'actifs.

De manière préférée, une telle composition ou un tel complément alimentaire comprend en outre de la vitamine D3 et de l'acétate de tocophérol.

Par taurine selon présente invention, on entend la taurine, l'hypotaurine, ou l'un de leurs sels. Dans la mesure où l'association selon l'invention est destinée à une mise en oeuvre par voie orale chez un individu, les sels pouvant être mis en oeuvre sont bien évidemment choisis pour leur totale innocuité. Conviennent à ce titre les sels alcalins ou alcalinoterreux, en particulier les sels de magnésium, manganèse, fer II ou zinc (II).

La teneur en taurine, hypotaurine ou l'un de ses sels, dans une composition cosmétique destinée à l'administration par voie orale ou dans un complément alimentaire conformes à l'invention, peut être comprise entre 1 et 40 % du poids total de la composition, notamment entre 5 et 40 % du poids total de la composition ou du complément, particulièrement entre 5 et 30 % du poids total de la composition ou du complément.

La teneur en taurine, hypotaurine ou l'un de ses sels, dans une composition cosmétique destinée à l'administration par voie orale ou dans un complément alimentaire conforme à l'invention, pour la voie orale, peut être telle que la dose journalière de ladite taurine, hypotaurine ou l'un de ses sels est comprise entre 4 et 700 mg/j, et notamment entre 40 et 300 mg/j.

L'invention concerne également un procédé cosmétique pour prévenir et/ou lutter contre la microinflammation à bas bruit des follicules pileux et/ou du scalp, permettant d'(de):

- améliorer la qualité de la chevelure ; et/ou
- lutter contre le vieillissement du cheveu chez l'homme de plus de 30 ans ou chez la femme ;

comprenant au moins l'administration, par voie orale, à un individu, d'une association d'actifs conforme à l'invention, ou d'une composition orale ou d'un complément alimentaire comprenant une telle association d'actifs.

Les procédés selon l'invention présentent les caractéristiques de procédés cosmétiques dans la mesure notamment où ils permettent d'améliorer l'esthétique de la chevelure, en particulier en luttant contre l'affinement progressif des cheveux apparaissant notamment avec l'âge. En outre, une association d'actifs, une composition ou un complément alimentaire selon l'invention peut être utilisé quotidiennement pendant plusieurs mois, sans prescription médicale. La présente invention se situe donc clairement en dehors du champ thérapeutique.

En particulier, les utilisations et/ou procédés cosmétiques de l'invention permettent de protéger les cheveux vieillis, notamment chez l'homme de plus de 30 ans ou chez la femme.

L'invention concerne également un complément alimentaire comprenant une partie des composés formant l'association d'actifs conforme à l'invention dans une première composition, et au moins l'autre partie des composés formant l'association d'actifs dans une deuxième composition, comme kit ou produit de combinaison pour une utilisation simultanée, séparée ou étalée dans le temps.

Il est entendu dans le cadre de la présente invention que « l'utilisation cosmétique par voie orale » recouvre l'utilisation de produits administrés par voie orale, ces produits étant par exemple sous forme de complément alimentaire comme exposé ci-après. Ces produits produisent un effet, au niveau de la chevelure, sur le plan esthétique et du confort, ou encore à visée beauté, par exemple en vue de la protéger, de la maintenir en bon état, et notamment de l'embellir, notamment par une augmentation du volume et de la tenue de la chevelure globale.

On entend par huile riche en acide pétrosélinique une huile comprenant au moins 20 % d'acide pétrosélinique et plus préférentiellement plus de 30 % d'acide pétrosélinique.

Alternativement, l'acide pétrosélinique, ou acide gras mono-insaturé (C18 :1 n-12 ou cis delta 6) ou acide delta-6-cis-octadecenoique en C18, est mis en oeuvre dans une association d'actifs conforme à l'invention.

Les ombellifères sont des plantes dont les fleurs sont disposées en ombelles. Les espèces particulièrement riches en acide pétrosélinique sont les Umbellifarea-Apiacea et Araliaceae. Les plantes du genre Thapsia sont également des sources d'acide pétrosélinique (Avato et al., Lipids, 2001, 36, 845). Les espèces de préférence utilisées dans l'invention sont le coriandre, le cerfeuil, la carotte, le céleri, le cumin, le carvi, le persil et l'aneth. L'huile d'ombellifère utilisée selon l'invention peut être extraite des graines de ces ombellifères, par exemple par broyage ou pressage, puis raffinage. L'huile d'ombellifère a une teneur en acide pétrosélinique qui varie selon la graine d'ombellifère dont elle est extraite. Pour une même ombellifère, la teneur en acide pétrosélinique varie aussi selon le pays d'origine de l'ombellifère et selon l'extraction qui peut être plus ou moins complète.

L'acide pétrosélinique est également un composé abondant (environ 48 %) de l'huile de graine de *Geranium sanguineum,* ainsi que de l'huile de graines de coriandre *Coriandrum sativum.*

Ainsi, selon un mode de réalisation, l'utilisation objet de la présente invention est telle que l'acide pétrosélinique est utilisé sous forme d'huile d'ombellifère ou de *Geranium sanguineum,* de préférence sous la forme d'huile de coriandre (*Coriandrum sativum*)*,* de cerfeuil, de carotte, de céleri, de cumin, de carvi, de persil ou d'aneth, de manière préférée sous la forme d'une huile de graines de coriandre (*Coriandrum sativum*).

Les teneurs en acide pétrosélinique sont variables selon que l'association d'actifs conformes à l'invention est mise en oeuvre dans une composition cosmétique destinée à une administration par voie orale ou dans un complément alimentaire.

Des teneurs sont indiquées ci-après à titre indicatif.

La teneur en acide pétrosélinique, dans une composition cosmétique destinée à l'administration par voie orale ou dans un complément alimentaire conforme à l'invention, peut être comprise entre 1 et 70 % du poids total, notamment entre 10 et 70 % du poids total, particulièrement entre 15 et 70 % du poids total de la composition ou du complément. La teneur en acide pétrosélinique dans une composition cosmétique destinée à l'administration par voie orale ou dans un complément alimentaire conformes l'invention, pour la voie orale, peut être telle que la dose journalière dudit acide pétrosélinique est comprise entre 5 et 1000 mg/j, et notamment entre 50 et 650 mg/j.

Selon une variante privilégiée, une composition cosmétique ou un complément alimentaire conformes à l'invention comprend en outre de la vitamine D3.

Selon une variante privilégiée, une composition cosmétique ou un complément alimentaire conformes à l'invention comprend l'association d'actifs et en outre et de la vitamine D3.

Selon une variante privilégiée, une composition cosmétique ou un complément alimentaire conformes à l'invention comprend en outre de l'acétate de tocophérol.

Selon une variante privilégiée, une composition cosmétique ou un complément alimentaire conformes à l'invention comprend en outre au moins un acide aminé choisi parmi les acides aminés constitutifs et/ou non constitutifs des protéines, en particulier l'arginine, la cystéine et/ou la méthionine.

Selon un mode de réalisation, la composition cosmétique destinée à une administration par voie orale ou un complément alimentaire conformes à l'invention comprend l'association d'actifs et du zinc, ou l'un de ses sels, de préférence du gluconate de zinc, l'acide pétrosélinique étant présent dans une teneur entre 20 et 70% en poids par rapport au poids total de ladite association d'actifs.

Par zinc, on entend le zinc ou l'un de ses sels (acétate, chlorure, citrate, lactate, gluconate, lactate, oxyde, carbonate ou sulfate de zinc), en particulier les sels de Zn (II), de préférence complexé par un ou plusieurs (poly)hydroxyacides tel que le gluconate.

Par (poly)hydroxyacide, on entend tout acide carboxylique qui comprend une chaine hydrocarbonée, linéaire ou ramifiée, saturée ou insaturée, de préférence saturée et/ou linéaire, comprenant de 1 à 10 atomes de carbone et de 1 à 9 groupes hydroxy, et comprenant de 1 à 4 groupes carboxyliques -C(O)-OH, dont au moins un desdits groupes -C(O)-OH est sous la forme carboxylate -C(O)-O-, complexée avec l'atome de Zn, de préférence Zn(II).

Plus particulièrement, le sel de zinc selon l'invention est complexé par deux groupes carboxylates tels que celui de Formule (I) :

$$R\text{-}C(O)\text{-}O\text{-}Zn\text{-}O\text{-}C(O)\text{-}R' \qquad (I)$$

dans laquelle R et R', identiques ou différents, représentent un groupe (C1-C6) (poly)hydroxyalkyle, ainsi que ses solvates, tels que les hydrates et leurs énantiomères.

De préférence, le composé de formule (I) est du gluconate de zinc.

Selon un mode de réalisation particulier de l'invention, le zinc n'est pas un oxyde de zinc mais un sel de zinc. Par Zn(II) on entend un atome de zinc de degré d'oxydation Zn2+.

Dans la mesure où le produit selon l'invention est destinée à une mise en oeuvre par voie orale chez un individu, les sels de zinc pouvant être mis en oeuvre sont bien évidemment choisis pour leur totale innocuité.

La teneur en zinc, en particulier en gluconate de zinc, dans une composition cosmétique destinée à l'administration par voie orale ou dans un complément alimentaire conforme à l'invention, peut être comprise entre 0,001 % et 30 % en poids, notamment entre 0,01 et 25 % en poids, particulièrement entre 0,1 et 20 % en poids du poids total de la composition ou du complément.

La teneur en gluconate de zinc, dans une composition cosmétique destinée à l'administration par voie orale ou dans un complément alimentaire conformes à l'invention, peut être telle que la dose journalière dudit gluconate de zinc est comprise entre 0,01 et 300 mg/j, notamment entre 0,1 et 200 mg/j, particulièrement entre 1 et 100 mg/j.

Selon un mode de réalisation préféré de l'invention, la composition cosmétique destinée à une administration par voie orale ou le complément alimentaire conformes à la présente invention, comprend de l'acide pétrosélinique, de la taurine et du zinc ou l'un de ses sels, de préférence complexé par un ou plusieurs (poly)hydroxyacides, en particulier du gluconate de zinc, l'acide pétrosélinique étant présent dans une teneur entre 20 et 70% en poids par rapport au poids total de ladite association d'actifs.

Selon un mode de réalisation, ladite composition cosmétique destinée à une administration par voie orale ou un complément alimentaire conformes à la présente invention comprend en outre à de la vitamine D3.

Selon un autre mode de réalisation préféré de l'invention, ladite composition cosmétique destinée à une administration par voie orale ou le complément alimentaire conformes à la présente invention comprend de l'acide pétrosélinique, de la taurine, du zinc ou l'un de ses sels, en particulier du gluconate de zinc, et de la vitamine D3.

Selon un mode de réalisation, ladite composition cosmétique destinée à une administration par voie orale ou un complément alimentaire conformes à la présente invention comprend en outre de l'acétate de tocophérol.

Selon un mode de réalisation, une composition cosmétique destinée à l'administration par voie orale ou un complément alimentaire conformes à l'invention peut en outre comprendre au moins une vitamine choisie parmi la vitamine B1, B5, B6, B8, B9, B12, C, D, et notamment D3, PP, ou le tocophérol (vitamine E) et ses dérivés, notamment ester tel que l'acétate, le succinate ou le palmitate de tocophérol, de préférence de l'acétate de tocophérol.

Selon un mode de réalisation, une composition cosmétique destinée à une administration par voie orale ou un complément alimentaire conformes à la présente invention comprend de préférence au moins de la vitamine E ou l'un de ses dérivés et/ou de la vitamine D, préférentiellement de la vitamine D3 et/ou de l'acétate de tocophérol.

Selon un mode de réalisation particulier, une composition cosmétique destinée à une administration par voie orale ou un complément alimentaire conformes à la présente invention comprend en outre la vitamine D3 et de l'acétate de tocophérol.

Ainsi, selon un mode de réalisation préféré de l'invention, la présente invention vise une composition cosmétique destinée à une administration par voie orale ou un complément alimentaire comprenant de l'acide pétrosélinique, de la taurine, du zinc ou l'un de ses sels, de préférence complexé par un ou plusieurs (poly)hydroxyacides, en particulier du gluconate de zinc, de la vitamine D3 et de l'acétate de tocophérol, l'acide pétrosélinique étant présent dans une teneur entre 20 et 70% en poids par rapport au poids total de ladite association d'actifs.

Les compositions selon l'invention peuvent en outre comprendre au moins un caroténoïde, notamment un caroténoïde choisi parmi le β-carotène, le lycopène, l'astaxanthine, la zéaxanthine et la lutéine, des flavonoïdes tels que les les anthocyanes, les flavonols, les flavanols (proanthocyanidines des extraits de raisin, catéchines des extraits de thé vert), les flavanones (hespéridine, diosmin), les acides phénoliques et dérivés (acide chlorogénique des extraits de café), les diterpènes, les stilbènes, les extraits de chicorés, des extraits de ginkgo biloba, des extraits de piment, des extraits de soja, d'autres sources de flavonoïdes possédant des propriétés antioxydantes, des acides gras, des prébiotiques, des probiotiques, du resveratrol, des acides aminés, du sélénium et des précurseurs de glutathion.

Les compositions orales ou les compléments alimentaires conformes à l'invention peuvent en outre comprendre au moins un probiotique, un prébiotique ou un mélange de probiotiques et un mélange de prébiotiques. A titre de microorganismes probiotiques, on peut notamment citer *Lactobacillus johnsonii* ou *Lactobacillus paracaseï.*

Les compositions cosmétiques destinées à une administration par voie orale ou compléments alimentaires conformes à la présente invention peuvent se présenter sous toutes les formes galéniques normalement utilisées pour la voie orale.

Dans la suite du présent texte, lorsqu'il est discuté des bornes x à y on entend les bornes x et y incluses.

Complément alimentaire

Selon un mode de réalisation, une composition cosmétique destinée à une administration par voie orale ou un complément alimentaire conformes à l'invention comprend :

(i) de l'acide pétrosélinique en une teneur comprise entre 20 et 70 % en poids, par rapport au poids total de l'association d'actifs ;

(ii) de la taurine en une teneur comprise entre 1 et 50 % en poids, notamment entre 5 et 40 % en poids, particulièrement entre 10 et 30 % en poids, par rapport au poids total de l'association d'actifs ; et/ou

(iii) au moins un (poly)hydroxyacide de zinc, de préférence du gluconate de zinc, en une teneur comprise entre 0,001 et 40 % en poids, notamment entre 0,01 et 25 % en poids, particulièrement entre 0,1 et 20 % en poids, par rapport au poids total de l'association d'actifs ;

(iv) optionnellement de la vitamine D3 en une teneur comprise entre 0,0001 et 1,0 % en poids, notamment entre 0,0001 et 0,5% en poids, particulièrement entre 0,0001 et 0,1 % en poids, par rapport au poids total de l'association d'actifs ; et/ou

(v) optionnellement de l'acétate de tocophérol en une teneur comprise entre 0,01 et 10 % en poids, notamment entre 0,1 et 10 % en poids, particulièrement entre 0,2 et 5 % en poids, par rapport au poids total de l'association d'actifs.

Selon un mode de réalisation particulier, une composition cosmétique destinée à une administration par voie orale ou un complément alimentaire conformes à l'invention comprend les ingrédients i) à v) ci-après, pris ensembles ou indépendamment :

(i) de l'acide pétrosélinique en une teneur comprise entre 1 et 70 % en poids, notamment entre 10 et 70 % en poids, particulièrement entre 15 et 70 % en poids, par rapport au poids total de la composition ou du complément ;

(ii) de la taurine en une teneur comprise entre 1 et 40 % en poids, notamment entre 5 et 40 % en poids, particulièrement entre 5 et 30 % en poids, par rapport au poids total de la composition ou du complément ; et/ou

(iii) au moins un (poly)hydroxyacide de zinc, de préférence du gluconate de zinc, en une teneur comprise entre 0,001 et 30 % en poids, notamment entre 0,01 et 25 % en poids, particulièrement entre 0,1 et 20 % en poids, par rapport au poids total de la composition ou du complément ;

(iv) optionnellement de la vitamine D3 en une teneur comprise entre 0,0001 et 1,0 % en poids, notamment entre 0,0001 et 0,5 % en poids, particulièrement entre 0,0001 et 0,1 % en poids, par rapport au poids total de la composition ou du complément ; et/ou

(v) optionnellement de l'acétate de tocophérol en une teneur comprise entre 0,01 et 10 % en poids, notamment entre 0,1 et 10 % en poids, particulièrement entre 0,2 et 5 % en poids, par rapport au poids total de la composition ou du complément.

Selon un mode de réalisation particulier, la composition cosmétique destinée à une administration par voie orale ou le complément alimentaire comprend l'ensemble des ingrédients (i) à (v) susmentionnés.

Une telle composition de type complément alimentaire ou une composition orale conformes à l'invention peut en particulier présenter les teneurs suivantes :

| Composants | % en poids par rapport au poids total de la composition |
|---|---|
| Acide pétrosélinique | 54,9 *(apporté par l'huile de graines de coriandre)* |
| Gluconate de zinc | 6,3 (dont 13,6 % de matière active) |
| Taurine | 18,7 (dont 98,5 % de matière active) |
| Vitamine E | 1,0 (dont 67 % de matière active) |
| Vitamine D3 | 0,03 (dont 2,5 % de matière active) |

Pour l'ingestion, de nombreuses formes de réalisation de compositions orales et notamment de compléments alimentaires sont possibles. Leur formulation est réalisée par les procédés usuels pour produire des dragées, gélules, gels, émulsions, comprimés, tablettes ou capsules molles.

Les compositions selon l'invention, destinées à une administration par voie orale, peuvent notamment comprendre l'intégralité ou une partie seulement de la dose journalière.

Autrement dit, une à trois compositions peuvent être administrées par jour.

Typiquement, la durée de ce traitement cosmétique destiné à une administration par voie orale peut être supérieure à 4 semaines, notamment de 4 à 24 semaines, avec le cas échéant une ou plusieurs périodes d'interruption.

Le complément alimentaire conforme à la présente invention peut comprendre une partie des actifs formant l'association selon l'invention dans une première composition et l'autre partie de ces actifs dans une deuxième composition, comme kit ou produit de combinaison pour une utilisation simultanée, séparée ou étalée dans le temps.

Ce complément peut être formulé de telle façon que les deux compositions sont sous les mêmes formes ou sous des formes différentes, par exemple choisies parmi celles citées plus haut. Un tel kit peut notamment être présenté dans un seul et même emballage.

Bien entendu l'homme du métier veillera à choisir les éventuels additifs complémentaires et/ou leur quantité de telle manière que les propriétés avantageuses de l'association selon l'invention ou de la composition comprenant l'association selon l'invention ne soient pas ou substantiellement pas altérées par l'adjonction envisagée.

Les actifs selon l'invention peuvent être formulés avec les excipients et composants usuels pour des compositions orales ou des compléments selon l'invention, à savoir notamment des composants gras et/ou aqueux, des agents humectants, des épaississants, des conservateurs, des agents de texture et/ou d'enrobage, des antioxydants, les arômes et des colorants usuels dans le domaine des complément alimentaire.

Comme actif cosmétique autre que l'association d'actifs conforme à l'invention, une composition ou un complément alimentaire conformes à l'invention peut contenir un actif additionnel hydrophile choisi parmi les sucres et les dérivés de sucre, les vitamines hydrosolubles, les extraits végétaux par exemple de romarin, d'écorce de pin ou de fruits comme l'orange, les peptides et acides aminés comme l'arginine, la méthionine, la cystéine, la citrulline et/ou un actif additionnel lipophile choisi parmi le rétinol (vitamine A) et ses précurseurs, notamment le Beta carotène, les caroténoïdes antioxydants comme le lycopène, la zeaxanthine, l'astaxanthine et la lutéine, les acides gras essentiels, les huiles végétales et animales notamment les huiles de poisson riches en oméga 3 essentielles, les phospholipides comme la lécithine, et leurs mélanges, les huiles de cucurbitacés comme la courge ou les sources d'enzymes antioxidantes comme le melon, les probiotiques et les prébiotiques, les vitamines et minéraux, les acides gras polyinsaturés et mono insaturées PUFA et MUFA et les oméga 3 et oméga 6.

D'autres caractéristiques et avantages de l'invention ressortiront mieux des exemples qui suivent, donnés à titre illustratif et non limitatif.

### Exemple 1

**Composition pour la voie orale sous forme de capsule molle.**

| Ingrédients | (mg/capsule molle) |
|---|---|
| Huile de graines de coriandre (65 % acide pétrosélinique) | 300 |
| Taurine | 76,10 |
| Gluconate de zinc | 25,75 |
| Vitamine E | 4,10 |
| Vitamine D3 | 0,115 |
| **Excipients** | |
| Huile de coco, raffinée | 112 |
| Cire d'abeille, jaune Cera flava | 22 |
| Lécithine de tournesol | 10 |
| **Capsule** | |
| Gélatine de poisson | 144,6 |
| Glycérol | 58,6 |
| Eau purifiée | 6,8 |

### Exemple 2

**Composition pour la voie orale sous forme de capsule molle.**

| Ingrédients | (mg/capsule molle) |
|---|---|
| Huile de graines de coriandre (65 % acide pétrosélinique) | 300 |
| Taurine | 76,10 |
| Vitamine E | 4,10 |
| **Excipients** | |
| Huile de coco, raffinée | 120 |
| Cire d'abeille, jaune Cera flava | 30 |
| Lécithine de tournesol | 12 |
| **Capsule** | |
| Gélatine de poisson | 160 |
| Glycérol | 58,6 |
| Eau purifiée | 6,8 |

### Exemple 3

**Composition pour la voie orale sous forme d'émulsion en stick.**

| Ingrédients | (g/stick) |
|---|---|
| Huile de graines de coriandre (dont 65 % d'acide pétrosélinique) | 0,40 |
| Taurine | 0,2 |
| Vitamine E | 0,0082 |
| **Excipients** | |
| Eau | 1,722 |
| Sucre | 0,911 |
| Fructose | 0,911 |
| Microcristalline cellulose | 0,032 |
| Carboxymethyl cellulose sodique | 0,004 |
| Mélange naturel de tocophérols | 0,034 |
| Huile de tournesol | 1,015 |
| Arôme naturel de citron | 0,034 |
| Sorbate de potassium | 0,013 |
| Acide citrique | 0,013 |
| Alginate de propylène glycol | 0,01 |

### Exemple 4

**Mise en évidence de l'effet d'une association d'acide pétrosélinique et de taurine sur la production d'une résolvine anti-inflammatoire endogène issue du métabolisme lipidique: la lipoxine A4 dans un modèle expérimental utilisant des cellules mononuclées humaines in vitro.**

Des cellules mononuclées sanguines sont mises en culture sous 5 % de $CO_2$ et à 37°C dans un milieu sans sérum pour macrophages (SFM Macrophage ; Invitrogen 12065074) pendant 24 heures. A l'issue de cette étape, le milieu est remplacé par le même milieu d'essai frais contenant en plus les actifs aux différentes doses pendant 30 minutes en présence des différents produits à évaluer (Huile de coriandre 0,25 mg/ml, taurine (3,1 mg/ml) La réponse inflammatoire a ensuite été déclenchée en présence de phorbol myristate (0,05 $\mu$M) et du calcium ionophore (1 $\mu$M) et d'un mélange de substrat lipidique composé d'acide docosahexaénoïque (DHA - 1 $\mu$g/mL) et d'acide eicosapentaénoïque (EPA - 1 $\mu$g/mL).

Les surnageants ont ensuite été prélevés au bout de 2 h de stimulation et congelés à -80°C avant préparation pour analyse en spectrométrie de masse.

Des triplicats expérimentaux (trois puits) ont été effectués par condition expérimentale. Dans chaque plaque de culture a été introduit un contrôle correspondant à des cellules stimulées par le mélange PMA/A23187 et/ou avec ajout du mélange équimolaire d'acides gras.

Les surnageants décongelés ont été concentrés par extraction en phase solide (SPE), repris dans du méthanol avant analyse spectométrique. La méthode analytique utilisée consiste à séparer les différents analytes par chromatographie liquide haute pression en fonction de leur temps de rétention et de les quantifier par spectrométrie de masse.

Les analyses ont été effectuées sur une chaine LC 1290 Infinity (Agilent Technologies) couplée à un spectromètre de masse 6460 Triple Quad LC/MS (Agilent Technologies) équipé d'une source d'ionisation en electrospray (Jet stream technology) opérant en monde négatif. Les séparations chromatographiques ont été réalisées sur une colonne ZorBAX SB-C18.

Les résultats on été obtenus en pg/mL de surnageant cellulaire. Ces données brutes ont été ensuite transformées par calcul pour obtenir le pourcentage d'activation (ou d'inhibition) par rapport au témoin de la plaque en utilisant le calcul suivant :

$$\% \text{ de modulation} = 100 \text{ x (valeur obtenue avec l'actif} - \text{valeur du}$$
$$\text{témoin)/valeur du témoin}$$

Ces pourcentages de modulation sont reportés dans le tableau ci-après.

Une association d'actifs conforme à l'invention comprenant de l'huile de coriandre, riche en acide pétrosélinique, et de la Taurine, ainsi que ces mêmes composés de façon individuelle, ont été testées conformément à ce qui est indiqué ci-dessus.

Les résultats obtenus à l'issu de ces tests comparatifs sont les suivants :

| Composés testés | Niveau de production de la lipoxine A4 |
|---|---|
| Huile de coriandre *(dont entre 60 et 75 % d'acide pétrosélinique)* 0,25 mg/ml | +19 % |
| Taurine 3,1 mg/ml | +38 % |
| Taurine 3,1 mg/ml + Huile de coriandre *(dont entre 60 et 75 % d'acide pétrosélinique)* 0,25 mg/ml | **+118 %** |

Dans ce cas également, on peut voir que l'effet d'une association conforme à l'invention sur la production de lipoxine A4 est très nettement supérieur à la somme des effets des composés mis en oeuvre individuellement.

En effet, on a pu observer une augmentation de la production de lipoxine A4 de 118 % par rapport au niveau basal de production de ce composant anti-inflammatoire lorsque les cellules lymphocytaires testées ont été mises en contact avec l'association d'actifs.

C'est donc bien un effet synergique d'une association d'actifs conforme à l'invention qui est ici observé et démontré.

**Revendications**

1. Utilisation cosmétique, par voie orale, de l'association d'acide pétrosélinique et de taurine, pour prévenir et/ou lutter contre la microinflammation à bas bruit des follicules pileux, permettant d'améliorer la qualité de la chevelure, en particulier chez l'homme de plus de 30 ans ou chez la femme, **caractérisée en ce que** l'amélioration de la qualité de la chevelure consiste en l'amélioration de la qualité de la fibre capillaire consistant en l'amélioration de la résistance à la traction des cheveux, et/ou au coiffage et/ou à la mise en forme des cheveux et/ou la prévention et/ou la lutte contre les cheveux mous et/ou cassants et/ou ternes et/ou fourchus et/ou fragilisés et/ou sensibilisés et/ou secs et/ou l'amélioration de la douceur et/ou de la vigueur des fibres capillaires.

2. Utilisation selon la revendication précédente, **caractérisée en ce que** l'acide pétrosélinique est utilisé sous forme d'huile d'ombellifère ou de *Geranium sanguineum,* de préférence sous la forme d'une huile de coriandre, de cerfeuil, de carotte, de céleri, de cumin, de carvi, de persil ou d'aneth, préférentiellement sous la forme d'une huile de graines de coriandre.

3. Utilisation selon l'une quelconque des revendications précédentes, **caractérisée en ce que** du gluconate de zinc est utilisé par voie orale, conjointement à ladite association.

4. Utilisation selon l'une quelconque des revendications précédentes, **caractérisée en ce que** de la vitamine D3 est utilisée par voie orale, conjointement à ladite association.

5. Utilisation selon l'une quelconque des revendications précédentes, **caractérisée en ce que** les actifs sont mis en oeuvre sous forme d'un complément alimentaire.

6. Utilisation selon la revendication 5, dans laquelle le complément alimentaire comprend en outre au moins une vitamine choisie parmi la vitamine B1, B5, B6, B8, B9, B12, C, D, de préférence D3, PP ou la vitamine E et ses dérivés, et comprend de préférence de la vitamine D et/ou de la vitamine E ou l'un de ses dérivés, préférentiellement de la vitamine D3 et/ou de l'acétate de tocophérol, plus préférentiellement de la vitamine D3 et de l'acétate de tocophérol.

**7.** Utilisation selon l'une quelconque des revendications 5 ou 6, dans laquelle le complément alimentaire comprend en outre au moins un acide aminé choisi parmi les acides aminés constitutifs et/ou non constitutifs des protéines, en particulier l'arginine, la cystéine et/ou la méthionine.

**8.** Utilisation selon l'une quelconque des revendications 5 à 7, dans laquelle le complément alimentaire comprend une association d'acide pétrosélinique, de taurine, et de zinc, ou l'un de ses sels, et de préférence du zinc complexé par un ou plusieurs (poly)hydroxyacides, en particulier le gluconate de zinc.

**9.** Utilisation selon l'une quelconque des revendications 5 à 8, dans laquelle le complément alimentaire comprend de l'acide pétrosélinique, de la taurine, du zinc ou l'un de ses sels, de préférence complexé par un ou plusieurs (poly)hydroxyacides, en particulier le gluconate de zinc, de la vitamine D3 et de l'acétate de tocophérol.

**10.** Complément alimentaire contenant une association d'acide pétrosélinique, de taurine, de zinc ou de l'un de ses sels, de préférence complexé par un ou plusieurs (poly)hydroxyacides, de préférence le gluconate de zinc, l'acide pétrosélinique étant présent dans une teneur comprise entre 20 et 70 % en poids par rapport au poids total de ladite association d'actifs.

**11.** Complément alimentaire selon la revendication 10, comprenant en outre de la vitamine D3 et de l'acétate de tocophérol.

**12.** Complément alimentaire selon la revendication 10 ou 11, comprenant :

    (i) de l'acide pétrosélinique en une teneur comprise entre 1 et 70 % en poids, notamment entre 10 et 70 % en poids, particulièrement entre 15 et 70 % en poids, par rapport au poids total du complément ;
    (ii) de la taurine en une teneur comprise entre 1 et 40 % en poids, notamment entre 5 et 40 % en poids, particulièrement entre 5 et 30 % en poids, par rapport au poids total du complément ; et/ou
    (iii) au moins un (poly)hydroxyacide de zinc, de préférence du gluconate de zinc, en une teneur comprise entre 0,001 et 30 % en poids, notamment entre 0,01 et 25 % en poids, particulièrement entre 0,1 et 20 % en poids, par rapport au poids total du complément ; et/ou
    (iv) optionnellement de la vitamine D3 en une teneur comprise entre 0,0001 et 1 % en poids, notamment entre 0,0001 et 0,5 % en poids, particulièrement entre 0,0001 et 0,1 % en poids, par rapport au poids total du complément ; et/ou
    (v) optionnellement de l'acétate de tocophérol en une teneur comprise entre 0,01 et 10 % en poids, notamment entre 0,1 et 10 % en poids, particulièrement entre 0,2 et 5 % en poids, par rapport au poids total du complément.

**13.** Procédé cosmétique pour prévenir et/ou lutter contre la microinflammation à bas bruit des follicules pileux et/ou du scalp, permettant d'améliorer la qualité de la chevelure, en particulier chez l'homme de plus de 30 ans ou chez la femme, comprenant au moins l'administration, par voie orale, à un individu, d'un complément alimentaire tel que défini dans l'une quelconque des revendications 10 à 12, **caractérisée en ce que** l'amélioration de la qualité de la chevelure comprend améliorer la résistance à la traction des cheveux, et/ou au coiffage et/ou à la mise en forme des cheveux et/ou prévenir et/ou lutter contre les cheveux mous et/ou cassants et/ou ternes et/ou fourchus et/ou fragilisés et/ou sensibilisés et/ou secs et/ou améliorer la douceur et/ou la vigueur des fibres capillaires.

**Patentansprüche**

**1.** Kosmetische orale Verwendung der Kombination von Petroselinsäure und Taurin, zur Vorbeugung und/oder Bekämpfung der schleichenden Mikroinflammation von Haarfollikeln, durch die sich die Haarqualität verbessern lässt, insbesondere bei Männern über 30 Jahre oder bei Frauen, **dadurch gekennzeichnet, dass** die Verbesserung der Haarqualität darin besteht, die Qualität der Haarfaser zu verbessern, darin bestehend, die Reißfestigkeit des Haares zu verbessern und/oder das Frisieren des Haares und/oder die Formgebung des Haaares zu verbessern und/oder schlaffes und/oder brüchiges Haar und/oder glanzloses und/oder gespaltenes und/oder kraftloses und/oder empfindliches und/oder trockenes Haar zu verhindern und/oder zu bekämpfen und/oder die Schlaffheit und/oder Kräftigkeit der Haarfasern zu verbessern.

**2.** Verwendung nach vorangehendem Anspruch, **dadurch gekennzeichnet, dass** Petroselinsäure in Form von Doldenblütleröl oder *Geranium sanguineum,* vorzugsweise in Form von Koriander-, Kerbel-, Karotten-, Sellerie-, Kreuzkümmel-, Kümmel-, Petersilien- oder Dillöl, vorzugsweise in Form von Koriandersamenöl, verwendet wird.

3. Verwendung nach einem der vorangehenden Ansprüche, **dadurch gekennzeichnet, dass** Zinkgluconat oral in Verbindung mit der Kombination verwendet wird.

4. Verwendung nach einem der vorangehenden Ansprüche, **dadurch gekennzeichnet, dass** Vitamin D3 oral zusammen mit der Kombination verwendet wird.

5. Verwendung nach einem der vorangehenden Ansprüche, **dadurch gekennzeichnet, dass** die Wirkstoffe als Nahrungsergänzungsmittel verwendet werden.

6. Verwendung nach Anspruch 5, wobei das Nahrungsergänzungsmittel ferner mindestens ein Vitamin umfasst, ausgewählt aus B1, B5, B6, B8, B9, B12, C, D, vorzugsweise D3, PP oder Vitamin E und seinen Derivaten, und vorzugsweise Vitamin D und/oder Vitamin E oder eines seiner Derivate umfasst, vorzugsweise Vitamin D3 und/oder F-Tocopherolacetat, vorzugsweise Vitamin D3 und F-Tocopherolacetat.

7. Verwendung nach einem der Ansprüche 5 oder 6, wobei das Nahrungsergänzungsmittel ferner mindestens eine Aminosäure umfasst, ausgewählt aus den Aminosäuren, die die Proteine bilden und/oder nicht bilden, insbesondere Arginin, Cystein und/oder Methionin.

8. Verwendung nach einem der Ansprüche 5 bis 7, wobei das Nahrungsergänzungsmittel eine Kombination aus Petroselinsäure, Taurin und Zink oder einem ihrer Salze und vorzugsweise mit einer oder mehreren (Poly)hydroxysäuren, insbesondere Zinkgluconat, komplexiertes Zink umfasst.

9. Verwendung nach einem der Ansprüche 5 bis 8, wobei das Nahrungsergänzungsmittel Petroselinsäure, Taurin, Zink oder eines ihrer Salze umfasst, vorzugsweise komplexiert mit einer oder mehreren (Poly)hydroxysäuren, insbesondere Zinkgluconat, Vitamin D3 und Tocopherolacetat.

10. Nahrungsergänzungsmittel, enthaltend eine Kombination von Petroselinsäure, Taurin, Zink oder einem ihrer Salze, vorzugsweise komplexiert mit einer oder mehreren (Poly)hydroxysäuren, vorzugsweise Zinkgluconat, wobei Petroselinsäure in einem Gehalt von 20 bis 70 Gew.-%, bezogen auf das Gesamtgewicht der Kombination von Wirkstoffen, vorhanden ist.

11. Nahrungsergänzungsmittel nach Anspruch 10, ferner umfassend Vitamin D3 und Tocopherolacetat.

12. Nahrungsergänzungsmittel nach Anspruch 10 oder 11, umfassend:

    i) Petroselinsäure mit einem Gehalt von 1 und 70 Gew.-%, insbesondere von 10 und 70 Gew.-%, insbesondere von 15 und 70 Gew.-%, bezogen auf das Gesamtgewicht des Ergänzungsmittels;
    ii) Taurin in einem Gehalt von 1 bis 40 Gew.-%, insbesondere 5 bis 40 Gew.-%, insbesondere 5 bis 30 Gew.-%, bezogen auf das Gesamtgewicht des Ergänzungsmittels; und/oder
    (iii) mindestens eine Zink(poly)hydroxysäure, vorzugsweise Zinkgluconat, in einem Gehalt von 0,001 bis 30 Gew.-%, insbesondere von 0,01 bis 25 Gew.-%, insbesondere von 0,1 und 20 Gew.-%, bezogen auf das Gesamtgewicht des Ergänzungsmittels; und/oder
    iv) gegebenenfalls Vitamin D3 in einem Gehalt von 0,0001 bis 1 Gew.-%, insbesondere von 0,0001 bis 0,5 Gew.-%, insbesondere von 0,0001 bis 0,1 Gew.-%, bezogen auf das Gesamtgewicht des Ergänzungsmittels; und/oder
    (v) gegebenenfalls Tocopherolacetat in einem Gehalt von 0,01 bis 10 Gew.-%, insbesondere von 0,1 bis 10 Gew.-%, insbesondere von 0,2 bis 5 Gew.-%, bezogen auf das Gesamtgewicht des Ergänzungsmittels.

13. Kosmetisches Verfahren zur Vorbeugung und/oder Bekämpfung der schleichenden Mikroinflammationen der Haarfollikel und/oder der Kopfhaut, durch das sich die Haarqualität verbessern lässt, insbesondere bei Männern über 30 Jahre oder bei Frauen, umfassend zumindest die orale Verabreichung an eine Person eines Nahrungsergänzungsmittels nach einem der Ansprüche 10 bis 12, **dadurch gekennzeichnet, dass** die Verbesserung der Qualität des Haares die Verbesserung der Reißfestigkeit des Haares und/oder der Frisier- und/oder Formgebungsbeständigkeit des Haares und/oder die Vorbeugung und/oder Bekämpfung von schlaffem und/oder sprödem Haar und/oder kraftlosem und/oder empfindlichem und/oder brüchigem und/oder sensibilisiertem und/oder trocknem Haar und/oder die Verbesserung der Geschmeidigkeit und/oder Kräftigkeit der Haarfasern umfasst.

**Claims**

1. Oral cosmetic use of the combination of petroselinic acid and taurine, to prevent and/or fight against low-level microinflammation of hair follicles, to improve the quality of the hair, in particular of a man over 30 years of age or a woman, **characterized in that** the improvement of the quality of the hair consists in the improvement of the quality of the hair fiber comprising the improvement of the tensile strength of the hair, and/or resistance to styling and/or shaping of the hair and/or preventing and/or combating limp and/or breakable and/or dull and/or split and/or embrittled hair and/or sensitized and/or dry and/or improving the softness and/or vigor of the hair fibers.

2. Use according to the preceding claim, **characterized in that** the petroselinic acid is used in the form of umbellifera plant oil or *Geranium sanguineum,* preferably in the form of coriander, chervil, carrot, celery, cumin, caraway, parsley or dill oil, preferably in the form of oil of coriander seeds.

3. Use according to any one of the preceding claims, **characterized in that** the combination of active agents taken orally also comprises zinc gluconate.

4. Use according to any one of the preceding claims, **characterized in that** the combination of active agents taken orally also contains vitamin D3.

5. Use according to any one of the preceding claims, **characterized in that** the active agents are used in the form of a food supplement.

6. Use according to claim 5, wherein the food supplement also comprises at least one vitamin chosen from vitamin B1, B5, B6, B8, B9, B12, C, D, preferably D3, PP or vitamin E and derivatives thereof, and preferably comprises vitamin D and/or vitamin E or a derivative thereof, preferably vitamin D3 and/or tocopheryl acetate, more preferably vitamin D3 and tocopheryl acetate.

7. Use according to any one of the claims 5 or 6, wherein the food supplement also comprises at least one amino acid chosen from amino acids that are and/or are not constituents of proteins, in particular arginine, cysteine and/or methionine.

8. Use according to any one of the claims 5 to 7, wherein the food supplement comprises a combination of petroselinic acid, taurine and zinc, or a salt thereof, and preferably zinc complexed with one or more (poly)hydroxy acids, in particular zinc gluconate.

9. Use according to any one of the claims 5 to 8, wherein the food supplement comprises petroselinic acid, taurine, zinc or a salt thereof, preferably zinc complexed with one or more (poly)hydroxy acids, in particular zinc gluconate, vitamin D3 and tocopheryl acetate.

10. Food supplement containing a combination of petroselinic acid, taurine, zinc or a salt thereof, preferably complexed with one or more (poly)hydroxy acids, preferably zinc gluconate, the petroselinic acid being present in a content of between 20% and 70% by weight relative to the total weight of the combination of active agents.

11. Food supplement according to claim 10, also comprising vitamin D3 and tocopheryl acetate.

12. Food supplement according to claim 10 or 11, comprising:

(i) petroselinic acid in a content of between 1% and 70% by weight, especially between 10% and 70% by weight, and particularly between 15% and 70% by weight, relative to the total weight of the supplement;
(ii) taurine in a content of between 1% and 40% by weight, especially between 5% and 40% by weight, and particularly between 5% and 30% by weight, relative to the total weight of the supplement; and/or
(iii) at least one zinc (poly)hydroxy acid, preferably zinc gluconate, in a content of between 0.001% and 30% by weight, especially between 0.01% and 25% by weight, and particularly between 0.1% and 20% by weight relative to the total weight of the supplement; and/or
(iv) optionally vitamin D3 in a content of between 0.0001% and 1% by weight, especially between 0.0001% and 0.5% by weight, and particularly between 0.0001% and 0.1% by weight relative to the total weight of the supplement; and/or

(v) optionally tocopheryl acetate in a content of between 0.01% and 10% by weight, especially between 0.1% and 10% by weight, and particularly between 0.2% and 5% by weight, relative to the total weight of the supplement.

13. Cosmetic process for preventing and/or combating low-level microinflammation of hair follicles and/or scalp to improve the quality of the hair, in particular of a man over 30 years of age or a woman, comprising at least the step of orally administering to an individual, a food supplement as defined in any one of claims 10 to 12, **characterized in that** the improvement of the quality of the hair comprises the improvement of the tensile strength of the hair, and/or styling and/or shaping of the hair and/or preventing and/or combating limp and/or breakable and/or dull and/or split and/or embrittled and/or sensitized and/or dry hair and/or improving the softness and/or vigor of the hair fibers.

## RÉFÉRENCES CITÉES DANS LA DESCRIPTION

**Documents brevets cités dans la description**

- WO 2004000293 A
- WO 2008071897 A

**Littérature non-brevet citée dans la description**

- **MAHÉ et al.** Androgenetic alopecia and micro inflammation. *Int. J. Dermatol.,* 2000, vol. 39, 576-584
- **TRUEB.** *Clinical intervention in aging hair,* 2006, vol. 1 (2), 121-129
- **MAHÉ et al.** *International Journal of Dermatology,* 2000, vol. 39, 576-584
- **AVATO et al.** *Lipids,* 2001, vol. 36, 845